# EUROPEAN PATENT APPLICATION

(11) **EP 2 160 973 A1**
(43) Date of publication of application: **10.03.2010**
(21) Application number: 08163781.1
(22) Date of filing: 05.09.2008
(51) Int. Cl.: A61B 5/00, G01N 33/487, H01M 10/00

(54) **Rechargeable biosensor**

(71) Applicant: Visgeneer, Inc., Hsinchu City 300 (TW)
(72) Inventor: Dai, Ken-Shwo, 300, Hsinchu City (TW); Wang, Yi-Kai, 300, Hsinchu City (TW)
(74) Representative: Epping - Hermann - Fischer

(57) **Abstract**

A rechargeable biosensor for the quantitative determination of an analyte is disclosed. The rechargeable biosensor mainly comprises a charging circuit, a charge storage circuit, and an operation circuit. The charging circuit may be selectively electrically coupled to an external power source for receiving a voltage; the charge storage circuit is electrically coupled to the charging circuit and being rechargeable by the charging circuit when the charging circuit is electrically coupled to the external power source; the operation circuit is electrically coupled to the charge storage circuit for receiving an operation voltage from the charge storage circuit, wherein the operation circuit is adapted to measure an electrical signal derived from the analyte and output a quantitative property of the analyte.

## Description

### FIELD OF THE INVENTION

This invention relates generally to a biosensor for the quantitative determination of an analyte and more particularly to a biosensor with a rechargeable charge storage circuit.

### BACKGROUND OF THE INVENTION

Biological molecules play an important role in the normal functioning of human bodies, and many diseases are often accompanied by the abnormal quantitative variation of these molecules. Therefore, if the quantity (e.g. concentration) can be monitored, a determination of the physical condition may be made. For example, in the case of diabetes mellitus, patients may have a better understanding whether their blood sugar is well controlled by regularly measuring the concentration of glucose in their bodies.

Over the past years, various biosensors have been developed for quantitative determination of an analyte in samples. The analyte, which is usually biomolecule in liquid samples, may include without limitation glucose, uric acid, cholesterol, triglyceride, glutamyl oxaloacetic transaminase (GOT), and glutamyl pyrubic transaminase (GPT). For example, of the biosensors currently sold on the market, blood glucose monitoring systems have been used daily and proven to be an important tool for diabetic patients to improve their glycemic control. The technologies applied to the biosensor for the measurement of analyte concentration can be colorimetry or electrochemistry.

### SUMMARY OF THE INVENTION

Among various components in a biosensor, a power source like battery is an essential part for providing electric power for the proper operation of the daily used home-care device. When using the biosensor for quantitative determination of an analyte, many users have experienced the inconvenience of replacing the battery periodically. Before the replacement may be correctly carried out, users have to know exactly the type, size, lifespan, and/or performance of the battery used by the biosensor. Even more inconveniently, they also have to know the location where suitable batteries may be purchased. Besides, the location is sometimes inaccessible, which causes many problems when users are anxious to know their body condition.

During the replacement of batteries, users also need to know how to install them correctly. If the installation is wrongly done, the biosensor may not function properly and may even become broken. Moreover, users also have to know how to maintain the batteries so that malfunction caused by leakage may be prevented. Therefore, it is desirable to provide users who need to regularly monitor their physical condition with a rechargeable biosensor by which the testing may be performed in an easy and convenient way.

Accordingly, it is an object of the present invention to provide a biosensor with a rechargeable charge storage circuit.

One embodiment of the present invention is to provide a rechargeable biosensor for the quantitative determination of an analyte. The rechargeable biosensor mainly comprises a charging circuit, a charge storage circuit, and an operation circuit. The charging circuit may be selectively electrically coupled to an external power source for receiving a voltage; the charge storage circuit is electrically coupled to the charging circuit and being rechargeable by the charging circuit when the charging circuit is electrically coupled to the external power source; the operation circuit is electrically coupled to the charge storage circuit for receiving an operation voltage from the charge storage circuit, wherein the operation circuit is adapted to measure an electrical signal derived from the analyte and output a quantitative property of the analyte.

In accordance with a preferred embodiment of the present invention, the operation circuit comprises a programmable detecting circuit for measuring the electrical signal derived from the analyte; a microcontroller electrically coupled to the programmable detecting circuit for receiving and processing the electrical signal; and an output circuit electrically coupled to the microcontroller for receiving the processed electrical signal from the microcontroller and for outputting the quantitative property of the analyte in accordance with the processed electrical signal.

Another embodiment of the present invention is to provide a biosensor rechargeable by an external device. The biosensor comprises a programmable detecting circuit for measuring an electrical signal derived from an analyte, wherein the programmable detecting circuit comprises a rewritable medium containing upgradable data; a microcontroller electrically coupled to the programmable detecting circuit for receiving and processing the electrical signal; an output circuit electrically coupled to the microcontroller for receiving the processed electrical signal from the microcontroller and for outputting a quantitative property of the analyte in accordance with the processed electrical signal; a transmission circuit electrically coupled to the microcontroller, the transmission circuit being adapted to transmit the processed electrical signal to and receiving a voltage from the external device; and a charge storage circuit electrically coupled to the transmission circuit and being rechargeable by the external device.

In accordance with a preferred embodiment of the present invention, the transmission circuit comprises a Universal Serial Bus circuit, RS-232 or other signal communication interface from which the biosensor may receive a voltage and store it in the charge storage circuit.

Other objects, advantages, and novel features of the invention will become more apparent from the following detailed description when taken in conjunction with the accompanying drawings.

### BRIEF DESCRIPTION OF THE DRAWINGS

These and other objects and advantages of the present invention will become apparent from the following description of the accompanying drawings, which disclose several embodiments of the present invention. It is to be understood that the drawings are to be used for purposes of illustrations only, and not as a definition of the invention.

In the drawings, wherein similar reference numerals denote similar elements throughout the several views:
FIG. 1 is a block diagram of the biosensor according to one embodiment of the present invention.
FIG. 2 is a block diagram of the biosensor according to another embodiment of the present invention.

### DESCRIPTION OF EMBODIMENTS OF THE INVENTION

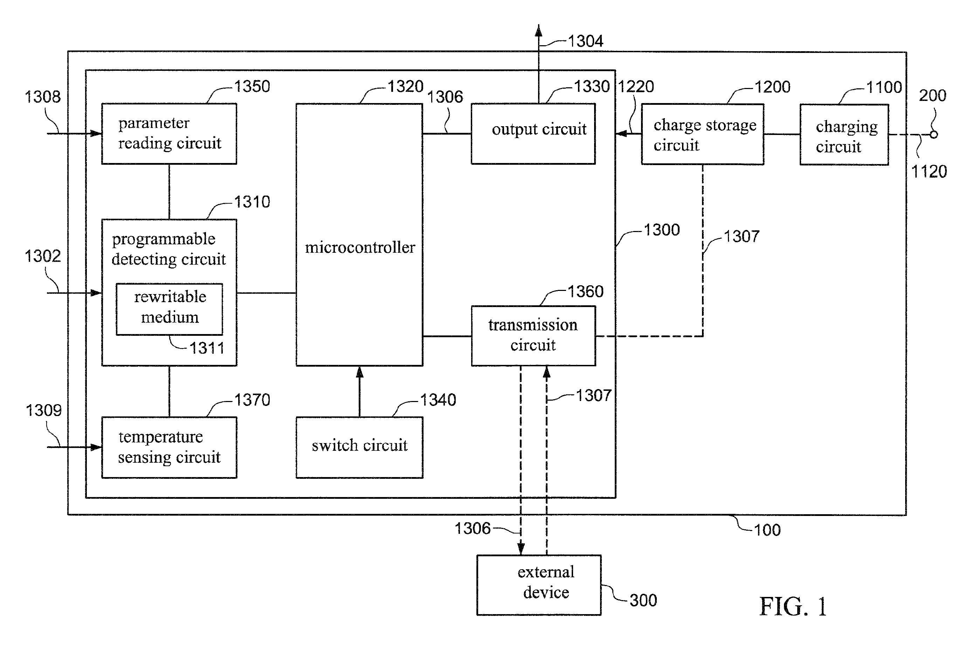

As shown in FIG. 1, one embodiment of the present invention discloses a rechargeable biosensor 100 which mainly comprises a charging circuit 1100, a charge storage circuit 1200, and an operation circuit 1300. The charging circuit 1100 may be selectively (i.e. not necessarily) electrically coupled to an external power source 200 for receiving a voltage 1120. The charge storage circuit 1200 is electrically coupled to the charging circuit 1100 and may be recharged by the charging circuit 1100 when the charging circuit 1100 is in electrical connection with the external power source 200; the operation circuit 1300, which is the unit adapted to carry out the quantitative determination of an analyte, is electrically coupled to the charge storage circuit 1200 for receiving an operation voltage 1220 from the charge storage circuit 1200, wherein the operation circuit 1300 is adapted to measure an electrical signal 1302 derived from the analyte and output a quantitative property 1304 of the analyte.

In a preferred embodiment, the charging circuit 1100 is capable of transforming the voltage 1120 received from the external power source 200. For example, it may transform the voltage 1120 into a direct voltage when the external power source 200 supplies an alternating voltage. Moreover, the charging circuit 1100 is also capable of adjusting the magnitude of the voltage 1120, e.g. lowering the voltage, to prevent the charge storage circuit 1200 from being damaged by the improper voltage.

In some embodiments, the charge storage circuit 1200 may comprise without limitation a built-in rechargeable battery, a capacitor, or any rechargeable charge storage device. With the integration of charge storage circuit 1200, the rechargeable biosensor 100 may be charged at any time when the external power source 200 (e.g. a power socket) is available, largely increasing its utility and making it more suitable for traveling users.

In accordance with a preferred embodiment of the present invention, the operation circuit 1300 may comprise a programmable detecting circuit 1310 for measuring the electrical signal 1302 derived from the analyte, a microcontroller 1320, and an output circuit 1330. The microcontroller 1320 is electrically coupled to the programmable detecting circuit 1310 for receiving and processing the electrical signal 1302. The output circuit 1330 is electrically coupled to the microcontroller 1320 for receiving the processed electrical signal 1306 therefrom and for outputting the quantitative property 1304 of the analyte in accordance with the processed electrical signal 1306.

In accordance with a preferred embodiment of the present invention, the programmable detecting circuit 1310 may comprise a rewritable medium 1311 containing upgradable data. With the incorporation of the rewritable medium 1311, users may upgrade the rechargeable biosensor 100 by simply transmitting data (e.g. a driver) of different versions into the rewritable medium 1311. Therefore, the necessity of changing a new biosensor 100 when users want to have a more powerful one may be obviated, and the expandability of the biosensor 100 of a preferred embodiment of the present invention may be enhanced.

The microcontroller 1320 of this invention, which can be implemented by any microcontroller available on the market, is a computer-on-a-chip. It may comprise without limitation a processor, memory, and input/output (I/O) ports. As the relevant knowledge of making and using microcontrollers is readily accessible in the art, further detailed description is omitted herein.

In accordance with a preferred embodiment of the present invention, the output circuit 1330 is capable of reporting to users the quantitative property 1304 (e.g. concentration) of the analyte under determination. In one embodiment of the present invention, the output circuit 1330 may show the result on a built-in screen of the rechargeable biosensor 100. In another embodiment of the present invention, the output circuit 1330 may comprise a speaker by which users are informed with the result by means of sound. Undoubtedly, other forms of outputting the quantitative property 1304 are also available to the present invention.

In addition to the units mentioned above, some preferred embodiments of this invention may optionally further comprise other units which, when incorporated, may provide the rechargeable biosensor 100 with synergistic effects.

As shown in FIG. 1, in one preferred embodiment, the operation circuit 1300 further comprises a switch circuit 1340, which is electrically coupled to the microcontroller 1320. In this embodiment, the switch circuit 1340 is adapted to induce the output circuit 1330 to output a predetermined voice instruction. For example, when triggered by users, the switch circuit 1340 may drive the output circuit 1330 to enunciate the steps of using the rechargeable biosensor 100. By the guidance of the predetermined voice instruction, users may learn to use the rechargeable biosensor 100 step-by-step without having to worry about breaking it.

Alternatively, depending on the way it is triggered, the switch circuit 1340 may also induce the output circuit 1330 to re-output the result of the determination or output other information.

In accordance with another preferred embodiment of the present invention, the operation circuit 1300 further comprises a parameter reading circuit 1350 electrically coupled to the programmable detecting circuit 1310. The parameter reading circuit 1350 is capable of receive a batch parameter 1308 usable by the programmable detecting circuit 1310 as a reference to measure the electrical signal 1302 derived from the analyte.

Conventionally, when a biosensor is to be used in the analyses of strips from different batches, users have to set the biosensor in accordance with the type of the strip to be used, which is complicated and time-consuming. With the incorporation of the parameter reading circuit 1350, the batch parameter 1308 of strips from different batches may be set within a short period of time by users with a parameter storage device (e.g. a code card). Therefore, when users want to make the quantitative determination of an analyte loaded on a strip taken from a specific batch, they can quickly set the batch parameter 1308 like the calibration parameter or strip type of the biosensor 100 by electrically coupling the parameter storage device to the parameter reading circuit 1350 (e.g. a code card reader).

In accordance with still another preferred embodiment of the present invention, the operation circuit 1300 further comprises a temperature sensing circuit 1370 electrically coupled to the programmable detecting circuit 1310. The temperature sensing circuit 1370 may sense ambient temperature 1309 of the biosensor 100, and the ambient temperature 1309 may be used by the programmable detecting circuit 1310 as a reference to measure the electrical signal 1302 derived from the analyte. Since the activity of the enzymes on the strip may vary with the temperature, the incorporation of the temperature sensing circuit 1370 may help improve the precision and accuracy of the quantitative determination.

In order to transmit the processed electrical signal 1306, either serial or parallel signal, to an external device 300 for follow-up processing, storage or representation, the operation circuit 1300 may further comprise a transmission circuit 1360. In accordance with still another preferred embodiment of the present invention, the transmission circuit 1360 comprises without limitation an RS-232 circuit or a Universal Serial Bus circuit, and the external device 300 may be but not limited to a personal computer, a personal digital assistant, a digital photo frame, or a digital camera.

In addition to the transmission of data, the transmission circuit 1360 may also transmit a voltage 1307 to the charge storage circuit 1200 for recharging it. As shown in FIG. 1, the transmission circuit 1360 may be electrically coupled to the charge storage circuit 1200 for directing a voltage 1307 received from the external device 300 thereto. By the aforementioned design, users may also use the external device 300 to recharge the charge storage circuit 1200 by setting up the electrical connection therebetween.

It should be emphasized that various units, including the switch circuit 1340, parameter reading circuit 1350, transmission circuit 1360, and temperature sensing circuit 1370, mentioned above are not necessary for the fundamental implementation of the embodiments of the present invention. However, in accordance with the preferred embodiments of the present invention, the incorporation of these units may largely enhance the utility of the invention and provide synergistic effects.

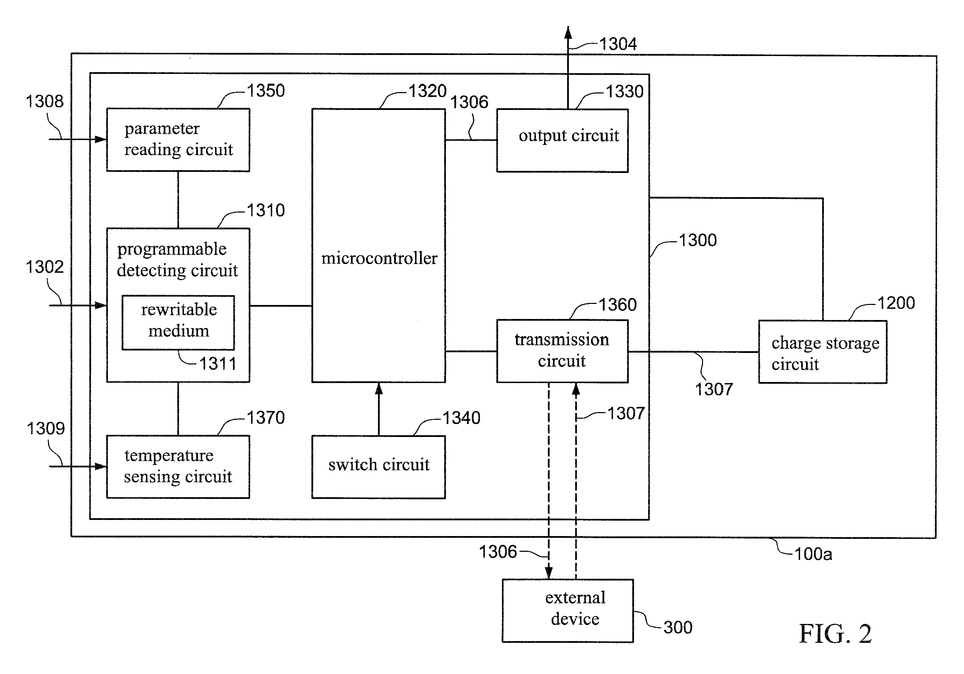

FIG. 2 is a configuration diagram of another embodiment of the present invention in which components similar to those of FIG. 1 are given similar identification numbers. The rechargeable biosensor 100a of this embodiment may comprise a programmable detecting circuit 1310 for measuring an electrical signal 1302 derived from an analyte, wherein the programmable detecting circuit 1310 comprises a rewritable medium 1311 containing upgradable data; a microcontroller 1320 electrically coupled to the programmable detecting circuit 1310 for receiving and processing the electrical signal 1302; an output circuit 1330 electrically coupled to the microcontroller 1320 for receiving the processed electrical signal 1306 from the microcontroller 1320 and for outputting a quantitative property 1304 of the analyte in accordance with the processed electrical signal 1306; a transmission circuit 1360 electrically coupled to the microcontroller 1320, the transmission circuit 1360 being adapted to transmit the processed electrical signal 1306 to and receiving a voltage 1307 from the external device 300; and a charge storage circuit 1200 electrically coupled to the transmission circuit 1360 and being rechargeable by the external device 300.

As shown in FIG. 2, the external device 300 is electrically coupled to the charge storage circuit 1200 via the transmission circuit 1360. Therefore, the charge storage circuit 1200 may receive the voltage via the transmission circuit 1360 from the external device 300 and recharged thereby when the electrical connection if formed between the biosensor 100a and the external device 300.

It will be understood that many other modifications can be made to the various disclosed embodiments without departing from the spirit and scope of the invention. For these reasons, the above description should not be construed as limiting the invention, but should be interpreted as merely exemplary of preferred embodiments.

## Claims

1. A rechargeable biosensor for the quantitative determination of an analyte, the rechargeable biosensor comprising:
a charging circuit selectively electrically coupled to an external power source for receiving a voltage;
a charge storage circuit electrically coupled to the charging circuit and being rechargeable by the charging circuit when the charging circuit is electrically coupled to the external power source; and
an operation circuit electrically coupled to the charge storage circuit for receiving an operation voltage from the charge storage circuit, wherein the operation circuit is adapted to measure an electrical signal derived from the analyte and output a quantitative property of the analyte.

2. The rechargeable biosensor as claimed in claim 1, wherein the operation circuit comprises:
a programmable detecting circuit for measuring the electrical signal derived from the analyte;
a microcontroller electrically coupled to the programmable detecting circuit for receiving and processing the electrical signal; and
an output circuit electrically coupled to the microcontroller for receiving the processed electrical signal from the microcontroller and for outputting the quantitative property of the analyte in accordance with the processed electrical signal.

3. The rechargeable biosensor as claimed in claim 2, wherein the programmable detecting circuit comprises a rewritable medium.

4. The rechargeable biosensor as claimed in claim 2, wherein the output circuit is capable of outputting the quantitative property of the analyte by means of sound.

5. The rechargeable biosensor as claimed in claim 4, wherein the operation circuit further comprises a switch circuit electrically coupled to the microcontroller, the switch circuit being adapted to induce the output circuit to output a predetermined voice instruction.

6. The rechargeable biosensor as claimed in claim 2, wherein the operation circuit further comprises a parameter reading circuit electrically coupled to the programmable detecting circuit, the parameter reading circuit being adapted to receive a batch parameter usable by the programmable detecting circuit as a reference to measure the electrical signal derived from the analyte.

7. The rechargeable biosensor as claimed in claim 2, wherein the operation circuit further comprises a transmission circuit electrically coupled to the microcontroller, the transmission circuit being adapted to transmit the processed electrical signal to an external device.

8. The rechargeable biosensor as claimed in claim 7, wherein the transmission circuit comprises an RS-232 circuit.

9. The rechargeable biosensor as claimed in claim 7, wherein the transmission circuit comprises a Universal Serial Bus circuit.

10. The rechargeable biosensor as claimed in claim 9, wherein the charging circuit is capable of receiving the voltage from the Universal Serial Bus circuit.

11. The rechargeable biosensor as claimed in claim 2, wherein the operation circuit further comprises a temperature sensing circuit electrically coupled to the programmable detecting circuit, the temperature sensing circuit being adapted to sense ambient temperature, which is usable by the programmable detecting circuit as a reference to measure the electrical signal derived from the analyte.

12. The rechargeable biosensor as claimed in claim 1, wherein the charge storage circuit comprises a battery.

13. A biosensor rechargeable by an external device, the biosensor comprising:
a programmable detecting circuit for measuring an electrical signal derived from an analyte, wherein the programmable detecting circuit comprises a rewritable medium containing upgradable data;
a microcontroller electrically coupled to the programmable detecting circuit for receiving and processing the electrical signal;
an output circuit electrically coupled to the microcontroller for receiving the processed electrical signal from the microcontroller and for outputting a quantitative property of the analyte in accordance with the processed electrical signal;
a transmission circuit electrically coupled to the microcontroller, the transmission circuit being adapted to transmit the processed electrical signal to and receiving a voltage from the external device; and
a charge storage circuit electrically coupled to the transmission circuit and being rechargeable by the external device.

14. The biosensor as claimed in claim 13, further comprising a switch circuit electrically coupled to the microcontroller, the switch circuit being adapted to induce the output circuit to output a predetermined voice instruction.

15. The biosensor as claimed in claim 13, further comprising a parameter reading circuit electrically coupled to the programmable detecting circuit, the parameter reading circuit being adapted to receive a batch parameter usable by the programmable detecting circuit as a reference to measure the electrical signal derived from the analyte.

16. The biosensor as claimed in claim 13, wherein the transmission circuit comprises a Universal Serial Bus circuit.

17. The biosensor as claimed in claim 13, further comprising a temperature sensing circuit electrically coupled to the programmable detecting circuit, the temperature sensing circuit being adapted to sense ambient temperature, which is usable by the programmable detecting circuit as a reference to measure the electrical signal derived from the analyte.

18. The biosensor as claimed in claim 13, wherein the charge storage circuit comprises a battery.
